# EUROPEAN PATENT SPECIFICATION

(11) **EP 4 294 289 B1**
(45) Date of publication and mention of the grant of the patent: **28.10.2026**
(21) Application number: 22718337.3
(22) Date of filing: 17.02.2022
(51) Int. Cl.: A61B 17/16, A61B 17/88

(54) **PROTECTED DRILL**
GESCHÜTZTE BOHRMASCHINE
FORET PROTÉGÉ

(30) Priority: 22.02.2021 US 202117181556
(43) Date of publication of application: 27.12.2023
(73) Proprietor: Warsaw Orthopedic, Inc., Warsaw, IN 46582 (US)
(72) Inventor: NAYET, Jerome, Minneapolis, Minnesota 55432 (US); JOSSE, Loic, Minneapolis, Minnesota 55432 (US)
(74) Representative: Viering, Jentschura & Partner mbB Patent- und Rechtsanwälte
(86) International application number: PCT/US2022/016809
(87) International publication number: WO 2022/178136

(56) References cited:
- EP-A2- 2 954 860
- US-A1- 2010 076 440
- US-A1- 2019 262 009

## Description

### FIELD

The present technology is generally related to a drill having a protected end for protecting patient tissue from a drill bit.

### BACKGROUND

The installation and insertion of bone screws in a patient poses many risks. At least one risk is the pre-operative step of drilling a passageway for a bone screw safely within a surgical opening of a patient, for example. Conventional drills may have sharp edges and a cutting tip that can cause accidental injuries to a patient. For example, in operation, an edge of a drill bit may catch an adjacent surface or "walk" away from an intended drill site and damage adjacent soft tissues. These problems may occur in all surgical settings requiring a drill although are particularly problematic in mini open surgeries and other minimally invasive surgical techniques, for example.

Further to the above, the US 2010/076440 A1 discloses a drill according to the preamble of claim 1.

Further surgical drills and screwdrivers as well related techniques are known from EP 2 954 860 A2 and US 2019/329388 A1

### SUMMARY

The present invention provides a drill as set out by claim 1. Further advantageous embodiments are described in the dependent claims.

In one aspect, a drill according to the invention is disclosed. The drill extends from a distal end to a proximal end and includes a housing extending in a longitudinal direction. The drill has a rotatable drive shaft including a first drive end disposed at the proximal end of the drill that is configured for coupling to a driver. The rotatable drive shaft has a main shaft portion extending in the longitudinal direction through the housing between the first drive end and a second drive end. The drill further includes an angled tip region defining the distal end of the drill, and the angled tip portion has a drill bit coupler configured to receive a drill bit and orient the drill bit in an angled direction with respect to the longitudinal direction thereby defining a drilling axis of the drill bit. The drill further includes a mechanism configured to transfer a rotational force applied to the first drive end through the second drive end and angled tip region to the drill bit coupler. The drill further includes a sleeve radially disposed at a distal end of the angled tip region and configured to radially surround at least a first portion of the drill bit when received in the drill bit coupler. According to the invention, the angled tip region further includes a compressible spring contacting the sleeve and configured to bias the sleeve in the angled direction, and the compressible spring is configured to surround at least a second portion of the drill bit when received in the drill bit coupler.

In another aspect, the disclosure provides for a positioning handle coupled to and disposed at a medial portion of the housing, for example.

In another aspect, the disclosure provides that the positioning handle may be angled with respect to the longitudinal direction and extend towards the proximal end of the drill, for example.

In another aspect, the disclosure provides that the sleeve has a conically tapered shape, for example.

In another aspect, the disclosure provides that in various embodiments, in a first mode of operation where the spring is in a neutral position, the sleeve and compressible spring are configured to surround lateral sidewalls of the drill bit when received in the drill bit coupler, for example.

In another aspect, the disclosure provides that in a second mode of operation, the compressible spring may be configured to compress in a direction parallel to the angled direction towards the mechanism, for example.

In another aspect, the disclosure provides that in a second mode of operation where the spring is in a neutral position, the compressible spring may be configured to compress in a direction parallel to the drilling axis towards the mechanism thereby exposing a tip of the drill bit for drilling, for example.

In another aspect, the disclosure provides that in the first mode of operation where the spring is in a neutral position, the sleeve and compressible spring completely surround the lateral sidewalls of the drill, for example.

In another aspect, the disclosure provides that in the first mode of operation where the spring is in a neutral position, a distal most end of the sleeve extends beyond the distal most end of the drill bit.

In another aspect, the disclosure provides for a flushing hole adjacent the angled tip region and including a flushing path to the mechanism.

In another aspect, the disclosure provides that the mechanism is a geared mechanism, for example. The geared mechanism may further include a first group of teeth disposed proximate the second drive end and a second group of teeth meshed with the first group of teeth and extending in a direction parallel with respect to the angled tip portion, for example. In various embodiments, the first group of teeth are meshed with the second group of teeth to thereby transfer a rotational force applied at the first drive end to the drill bit coupler, for example.

In another aspect, the disclosure provides for a manual hand driver configured to operably couple with the first drive end of the drive shaft, for example.

In another aspect, the disclosure provides for a powered driver configured to operably couple with the first drive end of the drive shaft, for example.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of an example drill in accordance with the principles of the present disclosure;
FIG. 2 is a side view of an example drill in accordance with the principles of the present disclosure;
FIG. 3 is a magnified view of the tip portion of an example drill in accordance with the principles of the present disclosure;
FIG. 4A is a top down view of a flushing portion of an example drill in accordance with the principles of the present disclosure;
FIG. 4B is a top down view of a flushing portion of an example drill with partially removed parts for ease of understanding in accordance with the principles of the present disclosure;
FIG. 5 is a side view of a gear mechanism in accordance with the principles of the present disclosure;
FIG. 6A is a top view of a gear mechanism in accordance with the principles of the present disclosure;
FIG. 6B is a bottom view of a gear mechanism in accordance with the principles of the present disclosure;
FIG. 7 is a cross sectional view of a gear mechanism in accordance with the principles of the present disclosure;
FIG. 8 is a perspective view of a portion of a gear mechanism and a drill bit in accordance with the principles of the present disclosure;
FIG. 9 is a perspective view of a portion of a gear mechanism and an aperture for receiving a drill bit in accordance with the principles of the present disclosure;
FIG. 10 is a side view of an example drill coupled to a powered driver in accordance with the principles of the present disclosure;
FIG. 11 is a side view of an example drill coupled to a manual hand driver in accordance with the principles of the present disclosure;
FIG. 12 is a side view of an example drill engaged with an aperture of an implant in accordance with the principles of the present disclosure;
FIG. 13A is a perspective view of an example implant having conically shaped apertures for seating a tip of a drill in accordance with the principles of the present disclosure;
FIG. 13B is an alternate perspective view of an example implant having conically shaped apertures for seating a tip of a drill in accordance with the principles of the present disclosure;
FIG. 14 is a perspective view of an example medical device that includes bone screw apertures that example drills of the present disclosure may progressively drive a bone screw through; and
FIG. 15 is an example method of operation of a medical device.

### DETAILED DESCRIPTION

As used herein, standard anatomical terms of location have their ordinary meaning as they would be understood by a person of ordinary skill in the art unless clearly defined or explained otherwise. It should be understood that various aspects disclosed herein may be combined in different combinations than the combinations specifically presented in the description and accompanying drawings. For example, characteristics of one embodiment may be combined or substituted with characteristics of another different embodiment unless those characteristics are clearly explained as being mutually exclusive. It should also be understood that, depending on the example, certain acts or events of any of the processes or methods described herein may be performed in a different sequence, may be added, merged, or left out altogether (e.g., all described acts or events may not be necessary to carry out the disclosed techniques and methods). In addition, while certain aspects of this disclosure are described as being performed by a single module, unit, or component for purposes of clarity, it should be understood that the techniques of this disclosure may be performed by a combination of units, modules, or components associated with, for example, a medical device such as a drill.

In some embodiments, the present disclosure is directed to a drill that is optimized for a medical setting and various types of surgical techniques, including anterior surgical techniques, lateral surgical techniques, and oblique surgical techniques. In some embodiments, a drill may be optimized to secure a spinal implant between adjacent vertebrae by securing at least one complimentary bone screw to the spinal implant and into an adjacent vertebrae. In some embodiments, and as mentioned above, the present disclosure may be employed in conjunction with spinal implants to treat spinal disorders such as, for example, degenerative disc disease, disc herniation, osteoporosis, spondylolisthesis, stenosis, scoliosis and other curvature abnormalities, kyphosis, tumor and fractures. In some embodiments, the present disclosure may be employed with other osteal and bone related applications, including those associated with diagnostics and therapeutics.

In some embodiments, the disclosed example drills may be alternatively employed in a surgical treatment with a patient in a prone or supine position, and/or employ various surgical approaches to the spine, including anterior, posterior, posterior mid-line, direct lateral, postero-lateral oblique, and/or antero lateral oblique approaches, and in other body regions. The present disclosure may also be alternatively employed with procedures for treating the lumbar, cervical, thoracic, sacral and pelvic regions of a spinal column. The drill of the present disclosure may also be used on animals, bone models and other non-living substrates, such as, for example, in training, testing and demonstration.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value and all numerical values therebetween. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior." Generally, similar spatial references of different aspects or components, *e.g*., a "proximal end" of one component and a "proximal end" of a different component, indicate similar spatial orientation and/or positioning, *i.e.,* that each "proximal end" is situated on or directed towards the same end of the device. Further, the use of various spatial terminology herein should not be interpreted to limit the various insertion techniques or orientations of the implant relative to the positions in the spine.

As used in the specification and including the appended claims, "treating" or "treatment" of a disease or condition refers to performing a procedure that may include administering one or more drugs, biologics, bone grafts (including allograft, autograft, xenograft, for example) or bone-growth promoting materials to a patient (human, normal or otherwise or other mammal), employing implantable devices, and/or employing instruments that treat the disease, such as, for example, micro-discectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (*e.g*., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, *e.g*., arresting its development, or relieving the disease, *e.g*., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

The components of disclosed embodiments described herein can be fabricated from biologically acceptable materials suitable for medical applications, including metals, synthetic polymers, ceramics and bone material and/or their composites. For example, the components of disclosed drills and bone screws, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (*e.g*., Nitinol, super elasto-plastic metals, such as GUM METAL^{®}), ceramics and composites thereof such as calcium phosphate (*e.g*., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaprolactone and their combinations.

Various components of disclosed embodiments may be formed or constructed of material composites, including but not limited to the above-described materials, to achieve various desired characteristics such as strength, rigidity, elasticity, compliance, biomechanical performance, durability and radiolucency or imaging preference. The components of expandable spinal implant system, individually or collectively, may also be fabricated from a heterogeneous material such as a combination of two or more of the above-described materials. The components of disclosed embodiments may be monolithically formed, integrally connected or include fastening elements and/or instruments, as described herein. For example, in some embodiments components comprising PEEK and/or titanium structures with radiolucent markers (such as tantalum pins and/or spikes) may be selectively placed on a drill, a drill bit, or a spinal implant, for example. In various embodiments, a drill is configured to bore into an adjacent vertebrae to provide a medical practitioner with a pilot hole or passageway for placement and/or sizing information to assist a surgeon with driving a a corresponding bone screw. The components of disclosed embodiments may be formed using a variety of subtractive and additive manufacturing techniques, including, but not limited to machining, milling, extruding, molding, 3D-printing, sintering, coating, vapor deposition, and laser/beam melting. Furthermore, various components of the expandable spinal implant system may be coated or treated with a variety of additives or coatings to improve biocompatibility, bone growth promotion or other features. For example, disclosed bone screws, may be selectively coated with bone growth promoting or bone ongrowth promoting surface treatments that may include, but are not limited to: titanium coatings (solid, porous or textured), hydroxyapatite coatings, or titanium plates (solid, porous or textured).

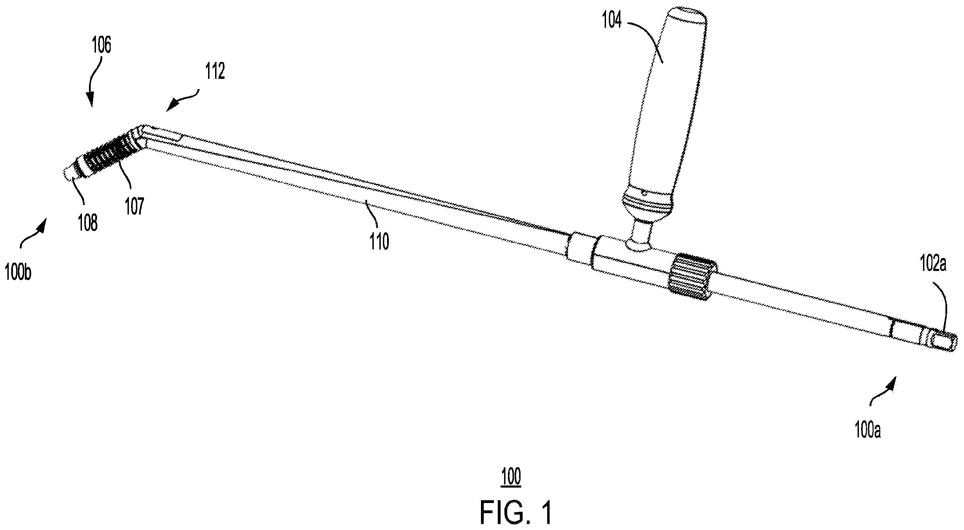

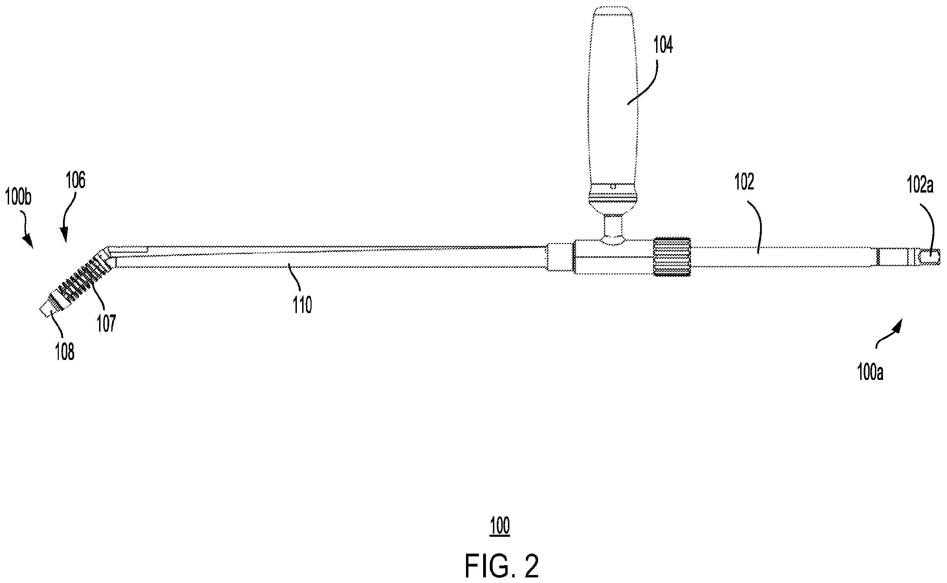

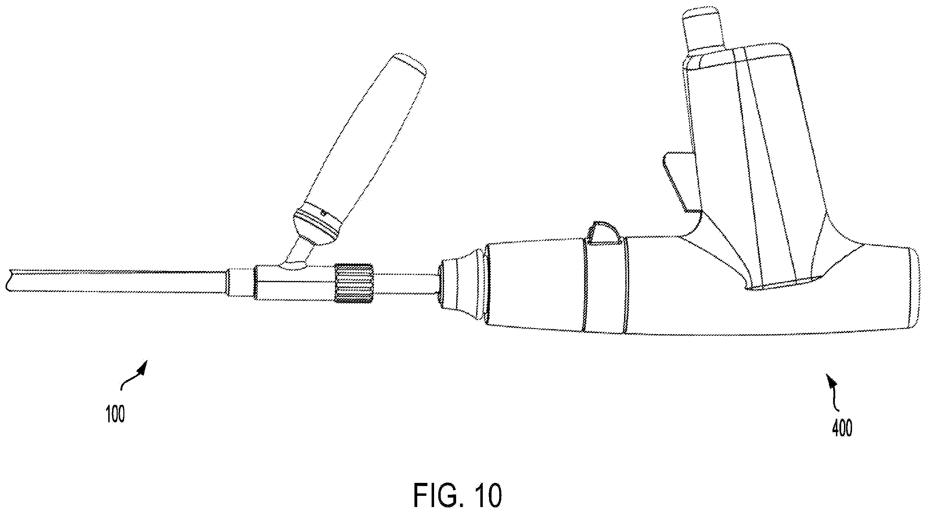

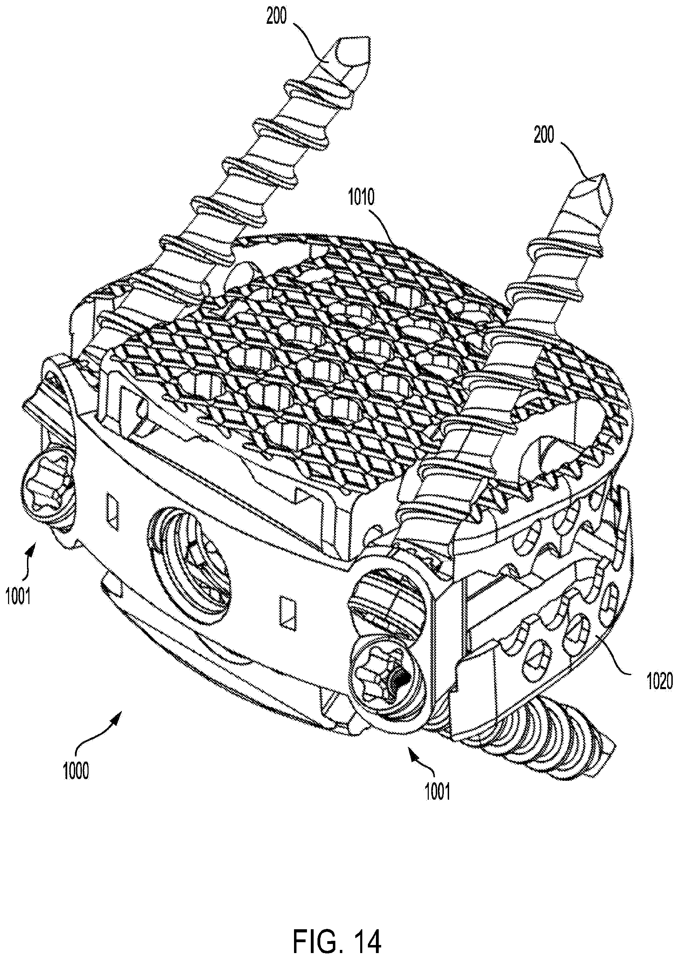

Referring generally to FIGS. 1-4B an example drill **100** is illustrated. FIG. 1 is a perspective view of an example drill **100** and FIG. 2 is a side view of the example drill **100.** Drill **100** may include a proximal end **100a** and a distal end **100b.** Drill **100** may also include a drive shaft **102,** a positioning handle **104,** a tip portion **106,** a spring **107,** a sleeve **108,** and a housing **110,** among other things. Drive shaft **102** may be configured to connect and disconnect with various types of drivers including manually operated handles and mechanically powered drive means that may be of a ratcheting or non-ratcheting type and which will be discussed in further detail below (*see, e.g.,* FIGS. 10-11). For example, drive shaft **102** may include a drive end **102a** and a main drive shaft portion **102b** extending in a longitudinal direction through a housing **110.** Drive end **102a** may comprise a variety of drive interfaces for coupling and uncoupling with various manually operated ratcheting handles and powered drivers, for example. Drive shaft **102** may freely rotate inside of housing **110** to transfer rotational force applied at the drive end **102a** at proximal end **100a** to drive end **102c** at distal end **100b,** for example. Positioning handle **104** may be securely held in place while drive shaft **102** freely rotates within housing **110.** Positioning handle **104** may be configured to assist with maintaining and controlling the drill **100,** *e.g.,* in view of torque transmitted through drive shaft and the corresponding resultant return forces. At least one advantage of positioning handle **104** is that a surgeon may have greater control maintaining drill **100** in a desired position while drilling a passageway (*e.g.,* a pilot hole) for a bone screw **200** (*see* FIG. 14). For example, when drilling a passageway for a bone screw into the anatomy of a patient a return force may apply a rotational force against the drill **100** and a surgeon may be able to maintain the drill **100** in the desired position, for example.

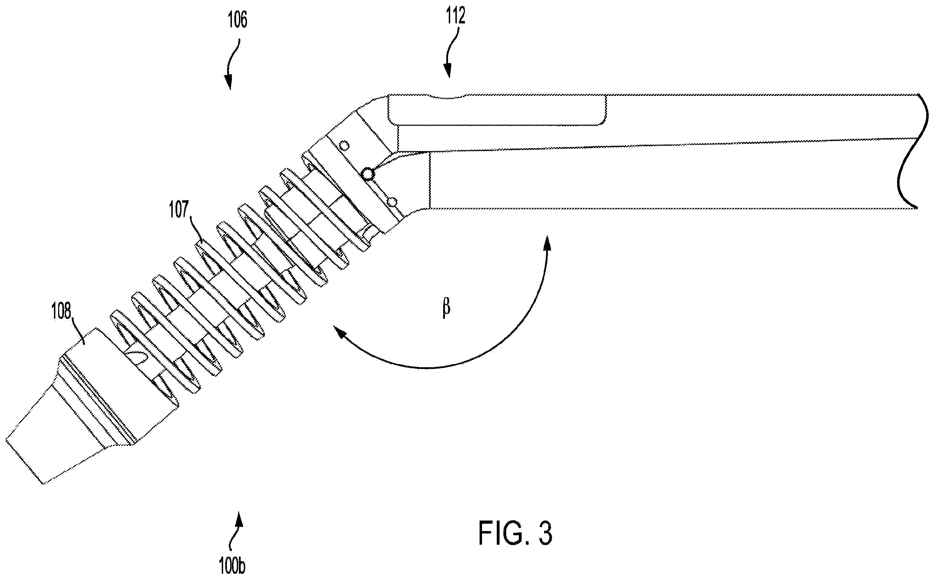

FIG. 3 is a magnified view of the tip portion **106** of an example drill **100** in accordance with the principles of the present disclosure. Tip portion **106** may be angled at a degree β (Beta) with respect to a longitudinal direction of housing **110** and/or drive shaft **102.** In various embodiments, tip portion **106** may extend in a direction that defines a drilling axis (rotation axis) of drill bit **109.** In some embodiments, tip portion **106** is angled such that the degree β corresponds to the ultimate desired angle of a passageway for receiving a bone screw **200.** In some embodiments, the degree β corresponds to an inclination of a bone screw aperture **1001** of a medical plate or medical device, *e.g*., medical device **1000** is a spinal implant including at least one bone screw aperture **1001** (*see* FIG. 23). In various embodiments, the tip portion **106** is angled to facilitate drilling of a passageway through a void space of a bone screw aperture **1001** of a spinal implant from a posterior approach while a patient is lying in a prone position, for example. Other surgical approaches, such as anterior, lateral, and/or posterior lateral approaches are also contemplated and may comprise adjustments to the degree β (Beta).

In various embodiments, tip portion **106** may be inclined about 20° - 60°, more particularly about 30° - 50°, and even more particularly about 40° - 45°, with respect to a longitudinal direction of housing **110.** However, it shall be understood that tip portion **106** may be angled at any degree β. Similarly, bone screw apertures **1001** may be angled at any degree with respect to endplates **1010, 1020** and tip portion **106** may be angled at a corresponding degree β to facilitate the installation of bone screw **200** therein (*see* FIG. 14). This angled arrangement may be advantageous for driving bone screw **200** while medical device **1000** is positioned between adjacent vertebral bodies. Furthermore, this angled arrangement may be advantageous to avoid anatomical landmarks and features such as the pelvic ring, rib cage, and iliac crest, of a patient, for example.

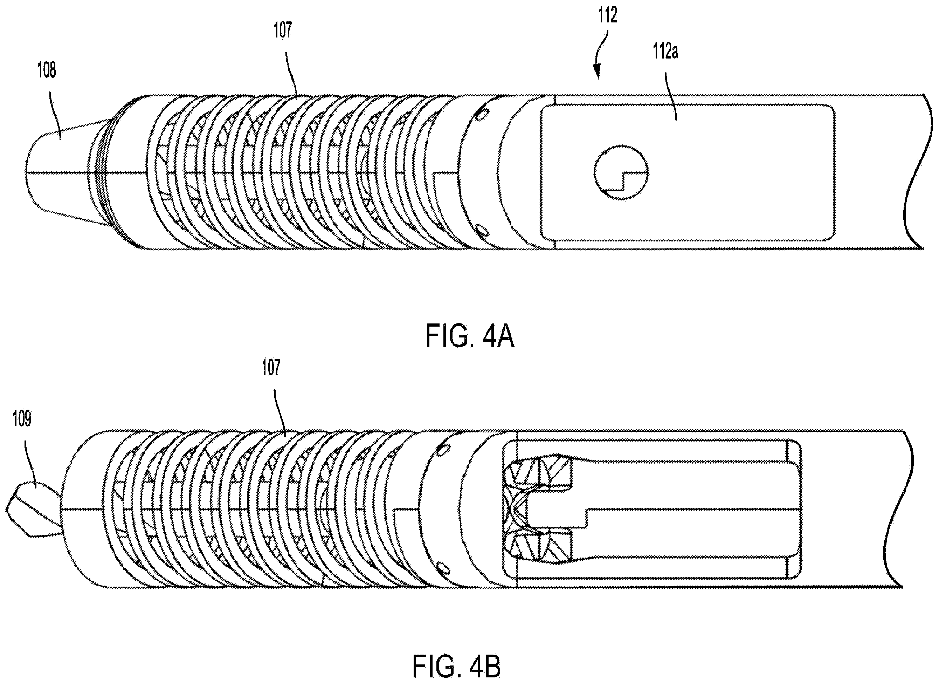

FIGS. 4A and 4B illustrate an example drill **100** that may include a flushing hole **112** having a flushing path to clean, lubricate, and/or inspect the components of tip portion **106.** For example, as shown in FIG. 4A a flushing hole **112** is shown, and in FIG. 4B a cover **112a** is removed to illustrate the flushing path. Flushing hole **112** may be advantageous for cleaning the interior orifices of tip portion **106.** Also shown in FIG. 4A is sleeve **108** which is a protective sleeve having a conical shape that surrounds drill bit **109.** For example, sleeve **108** may cover or surround drill bit **109** such that adjacent patient tissue is protected from drill bit **109.**

Sleeve **108** may be composed of elastomeric materials, thermoplastic materials, metallic materials, and various combinations thereof. In one embodiment, sleeve **108** is composed of an elastomeric material to provide flexibility and a high coefficient of friction for engaging and/or being seated within a bone screw aperture **1001,** for example. In an alternate embodiment, sleeve **108** is composed of metallic material, *e.g*., stainless steel and/or titanium. In another embodiment, sleeve **108** is composed of thermoplastic material, *e.g*., Polyether ether ketone (PEEK) and/or other organic thermoplastic polymers in, *e.g.,* the polyaryletherketone (PAEK) family. In another embodiment, sleeve **108** is composed of polyphenylsulfone (PPSU), also referred to as Radel by those with skill in the art. In another embodiment, sleeve **108** is composed of various combinations of the above enumerated materials. However, it shall be understood that the above enumerated materials are examples, and they shall not be construed as limiting.

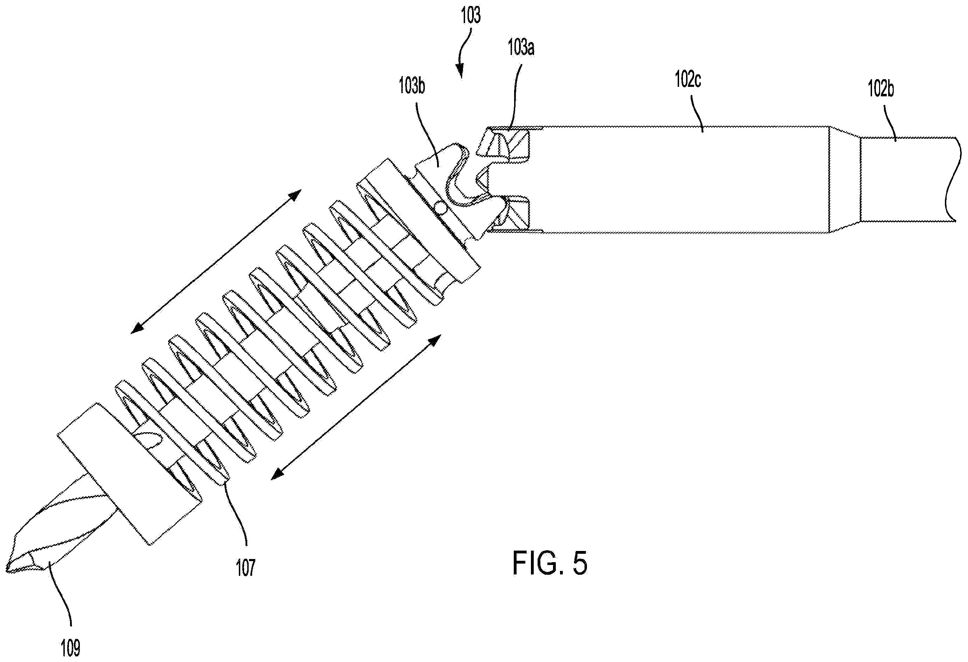

Referring generally to FIGS. 5-6B, operative characteristics of an example drill **100** will be explained. FIG. 5 illustrates a tip portion **106** (also referred to as an angled tip region) with a sleeve **108** and housing **110** removed for ease of explanation. In FIG. 5, it is shown that a spring **107** generally surrounds drill bit **109.** Spring **107** may be a compressible spring, a helical spring, a coil spring, of the like. In at least one embodiment, spring **107** takes the form of a compressible material such as rubber or foam. In some embodiments, spring **107** is covered by a protective cover to prevent debris and other foreign matter from entering in between the coils. In operation, a surgeon may press a proximal most end of sleeve **108** within a bone screw aperture **1001,** or alternatively against a surface to be drilled. In doing so, spring **107** may compress (shown by double sided arrows) and drill bit **109** may extend out of sleeve **108** (beyond sleeve **108).** For example, drill bit **109** is rigidly secured to drill **100** and sleeve **108** and spring **107** are movable with respect to drill bit **109.** At least one advantage of this arrangement, is that drill bit **109** may be protected and/or covered by sleeve **108** while drill bit **109** advances into a surface to be drilled and/or through bone screw aperture **1001.** For example, lateral sidewall surfaces of drill bit **109** are continuously protected from adjacent structures such as tissue while drill bit **109** continues to advance through sleeve **108** and into a boney structure.

Consistent with the disclosure herein, drill **100** may be understood as operating in various modes of operation. For example, a protected mode of operation and a drilling mode of operation. For example still, in a first mode of operation where the spring 107 is in a neutral position (non compressed position) the sleeve **108** and spring **107** cover and/or surround the lateral sidewalls of the drill bit **109,** for example. In a second mode of operation where the spring **107** is in a compressed or partially compressed position due to the sleeve **108** acting against a bearing or retaining surface, the spring **107** may be compressed in a direction parallel to an extension direction of drill bit **109,** for example. The extension direction of drill bit **109** may be coincident with a rotation axis of the drill bit **109** (drilling axis). Accordingly, in the second mode of operation, and due to the compression of spring **107,** drill bit **109** may move through the sleeve **108** thereby exposing a tip of the drill bit **109** for drilling, for example. Additionally, in various embodiments and in the first mode of operation, the sleeve **108** and compressible spring **107** completely surround the lateral sidewalls of the drill bit **109.** Furthermore, in various embodiments, and in the first mode of operation, a distal most end of the sleeve **108** extends distally farther than a distal most end of the drill bit **109.**

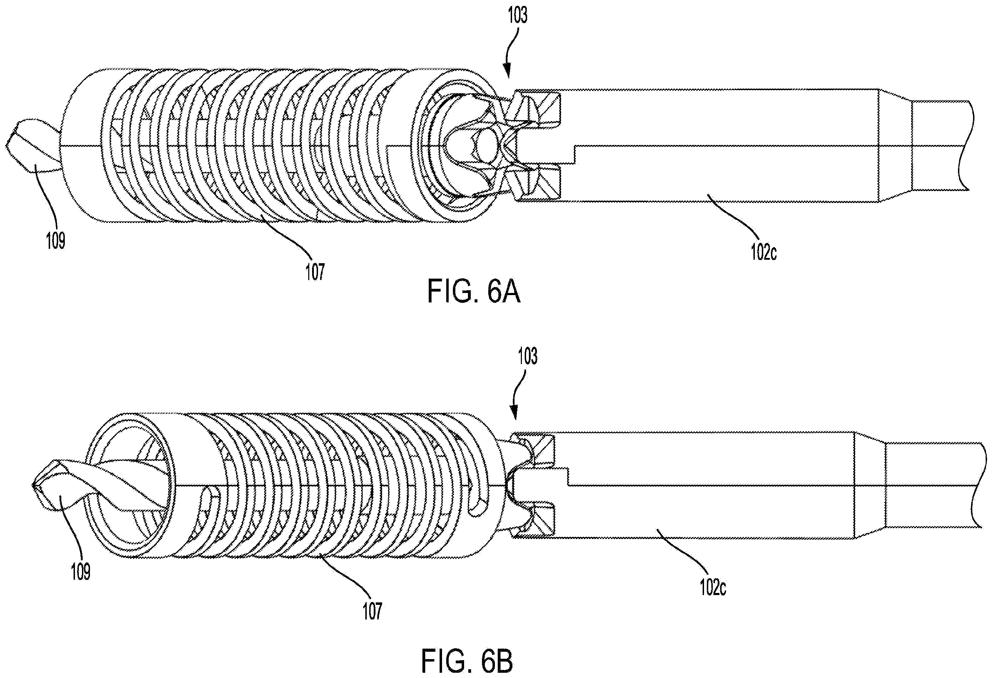

Also as shown in FIGS. 6-6b, an example gear mechanism **103** may be provided. Gear mechanism **103** may include worm gears, beveled gears, miter gears, planetary gears, sliding gears, helical or spiral gears, gear coupling parts, pawls, having teeth of various sizing and shapes for directing a rotation of the drive shaft **102** to drive end **102c.** For example, applying a rotation force at drive end **102a** may apply an equal or substantially equal rotation force at drill bit **109** because the gear mechanism **103** may redirect the rotation force. As illustrated, gear mechanism **103** may include a first body portion supporting a group of teeth **103a** that are meshed with a second group of teeth **103b** supported by a second body portion. In the example embodiment, the first group of teeth **103a** includes fourth teeth and the second group of teeth **103b** includes four teeth although the total number of teeth may be more or less. Those with skill in the art will readily appreciate that the particular geometry and number of teeth **103a, 103b** may be modified to accommodate any particular angle β *(see* FIG. 3). Additionally, in some embodiments, gear mechanism **103** may be designed to provide a mechanical advantage, such increasing or lowering the speed of rotation. For example, when a ratio of teeth sizing of teeth **103a, 103b** is inferior or superior with respect to the other.

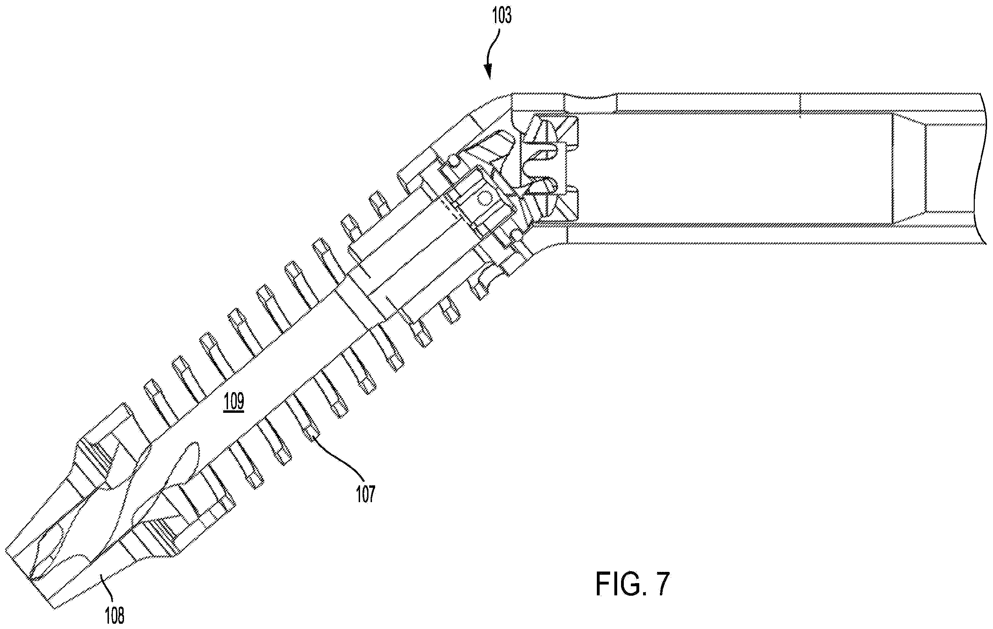

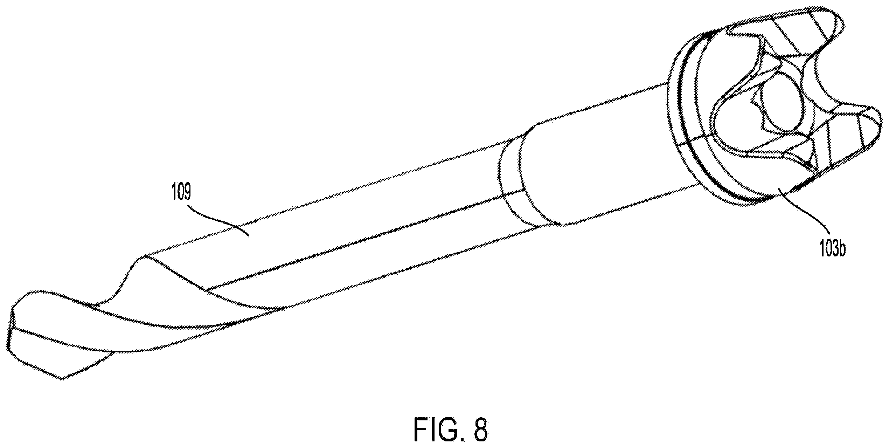

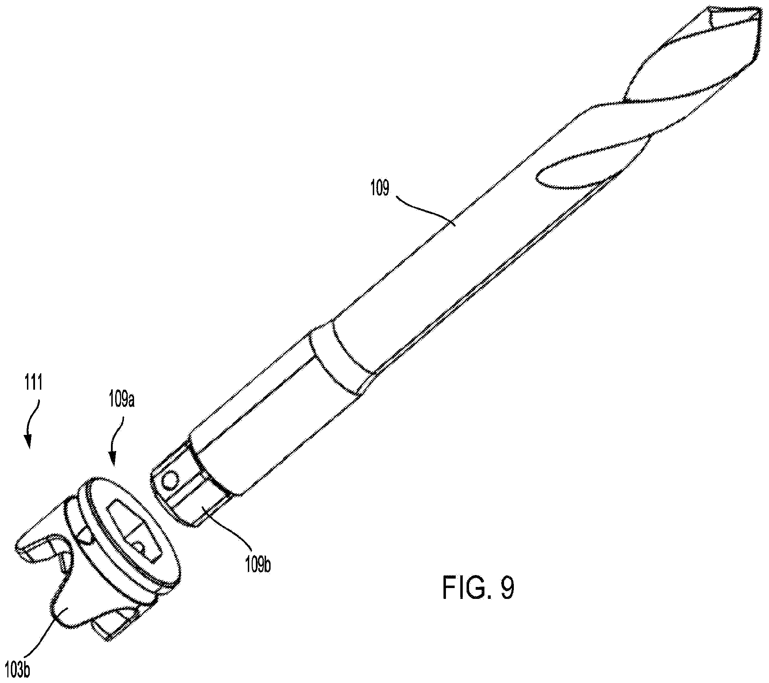

As illustrated in FIG. 7, a cross sectional view of spring **107,** sleeve **108,** drill bit **109,** and gear mechanism **103** is illustrated. In the cross sectional view, it is shown how drill bit **109** may rotate due to teeth **103a** being meshed with teeth **103b** while also being protected by spring **107** and sleeve **108.** As illustrated in FIG. 8, drill bit **109** may be coupled to an opposite side of the second body portion supporting teeth **103b,** for example. As illustrated in FIG. 9, a drill bit coupler **111** may comprise the second body portion and may include a drill bit aperture **109a** configured to receive a coupling end **109b** of drill bit **109** on one end and teeth 103b on an opposite end. In some embodiments, the drill bit coupler **111** may include an extension shaft (not illustrated). The drill bit aperture **109a** and coupling end **109b** of drill bit **109** may correspond in size and shape to one another. For example, drill bit aperture **109a** and coupling end **109b** may have a hex, hexalobular, square, star, torx, prismoidal, polygonal, etc. shape dimensioned such that coupling end **109b** may be seated firmly within drill bit aperture **109a.**

In an alternate embodiment, drill **100** may include a joint mechanism in lieu of gear mechanism **103** (not illustrated). For example, although not illustrated herein, the parent application from which this application is a continuation in part of, illustrates a joint mechanism **105** that may be substituted with gear mechanism **103.** For example, U.S. Pat. App. No. 17/123,906, discloses a joint mechanism **105** at FIG. 17 that may be operable/drivable via drive shaft **102** in the same, similar, or substantially the same way as gear mechanism **103** as explained above.

In an alternate embodiment, drill **100** may include a flexible shaft that may bend through the angled tip region in lieu of gear mechanism **103** and/or the joint mechanism as described above. For example, a flexible shaft mechanism may extend from the distal end **100a** to the angled tip region **106** where a drill bit **109** may be coupled to a distal end of the flexible shaft. For example still, the flexible shaft mechanism may comprise a first drive end **102a** and a second drive end **102b** comprising a drill bit coupler **111** or the like.

The described flexible shaft mechanism can be formed of an elastomeric and/or metallic material for example. In embodiments including metallic materials the flexible shaft mechanism may comprise an undulating pattern of transverse cuts or seams across the width of the flexible shaft mechanism that form flexible indentations enabling the flexibility of the described flexible shaft. For example, an undulating dove tail pattern, c-shaped pattern, webbed pattern, etc. For example still, the flexible shaft may be formed with a plurality of successive and organized cuts making the shaft flexible laterally although still strong in tension and sufficient to apply rotational forces to drill bit **109** similarly as explained herein. In at least one embodiment, the flexible shaft mechanism can be made of an assembly of springs. Additionally, the flexible shaft mechanism may extend longitudinally through housing **110** of drill **100** until a region approximately corresponding with drill bit coupler **111** and may include a drill bit coupler 111 and/or a similar aperture for receiving a drill bit **109** such as aperture **109a,** for example.

FIG. 10 illustrates an example drill **100** operably coupled to a powered driver 400 in accordance with the principles of the present disclosure. Powered driver **400** may be powered by any means, *e.g*., electrically operated or pneumatically operated. At least one example powered drill is the POWEREASE^{™} System sold by Medtronic and/or the powered rotary-type handpiece described in U.S. Pat. No. 10,456,122.

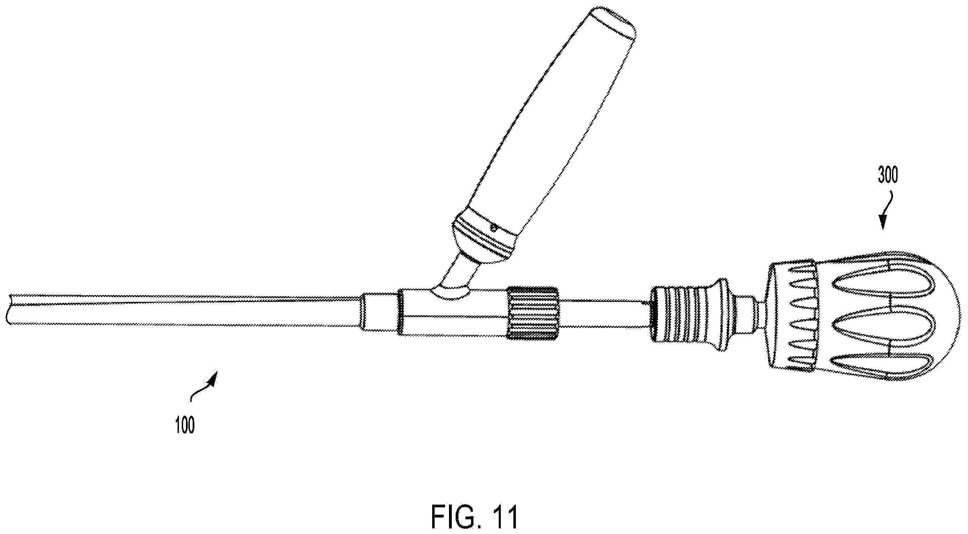

FIG. 11 illustrates an example drill **100** operably coupled to a manual hand driver **300** in accordance with the principles of the present disclosure. Hand driver **300** may selectively couple and uncouple with drive end **102a** of drive shaft **102.** At least one example of a manual hand driver **300** may be the commercially available QC handle sold by Medtronic of Minneapolis Minnesota. In various surgical techniques, a manual hand driver **300** as illustrated may be advantageous for performing gentle drilling, cleaning, excavation, and/or boring of a relatively soft or damaged bone, for example.

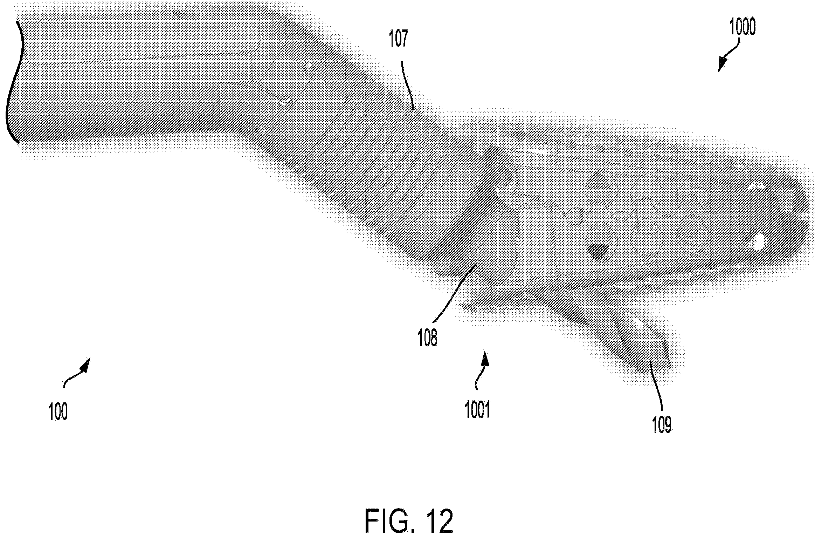

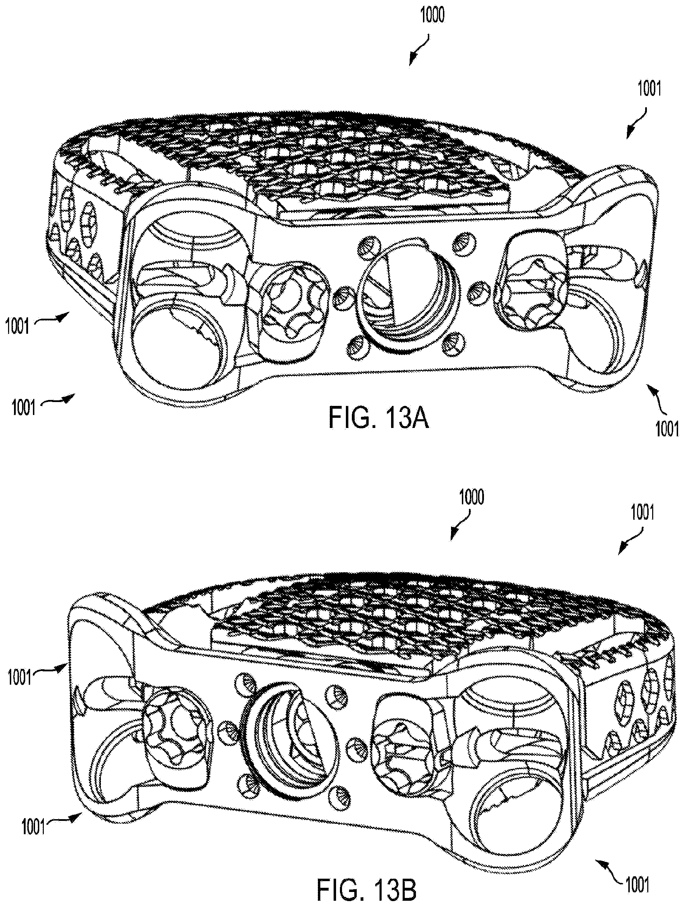

FIG. 12 illustrates an example drill **100** seated within a bone screw aperture **1001** of an implant **1000.** As illustrated, a conically shaped sleeve **108** is seated within a corresponding conically shaped bone screw aperture **1002** of implant **1000,** for example. For example, bone screw aperture **1002** may taper at the same or similar extend as sleeve **108** may taper and/or bone screw aperture **1002** may have a the same, similar, or substantially the same cross sectional dimensions. Drill **100** may be firmly pressed towards implant **1000** such that spring **107** is fully compressed thereby enabling drill bit **109** to extend through bone screw aperture **1001** beyond a bottom surface of a bottom endplate of implant **1000.** FIGS. 13A and 13B illustrate an alternate implant **1000** having four conically tapered bone screw apertures **1001.** FIG. 14 illustrates an example implant **1000** with four bone screws **200** extending through bone screw apertures **1001** illustrating that after drill bit **109** has bored various passageways within a boney structure the bone screws **200** may be installed in the same target trajectory.

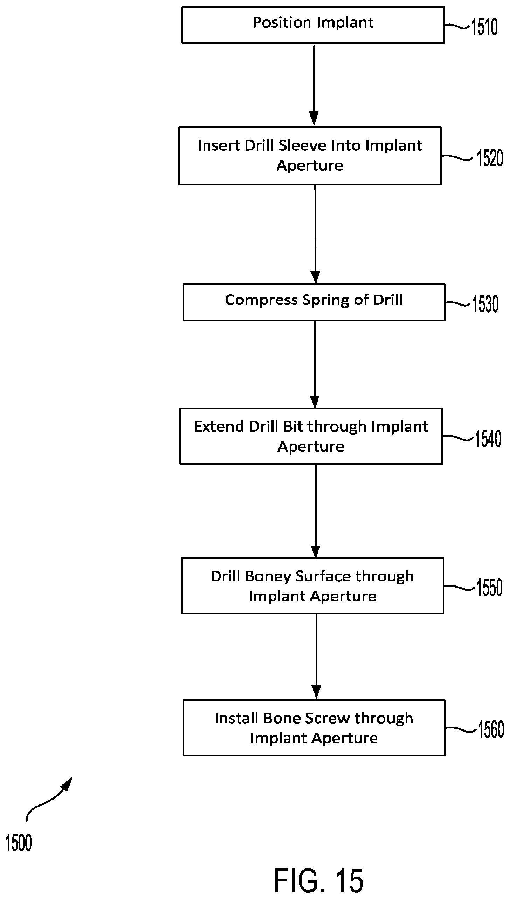

FIG. 15 illustrates an example method in accordance with the principles of the above disclosure, wherein said method does not form part of the invention as such, but is useful for understanding the claimed invention. The method may be implemented with various drill **100** embodiments disclosed hereinabove, for example. In practice, at step **1510** an end user such as a surgeon may position an implant between adjacent vertebral bodies. The end user may expand the implant such that it is relatively firmly positioned between the two vertebral bodies (*e.g.,* a superior vertebral body and an inferior vertebral body). At step **1520,** the end user may be provided with a protected drill as disclosed herein and insert a conically tapered protective sleeve portion into a bone screw aperture of the implant. At step **1530,** the end user may firmly press the drill against the implant thereby compressing a protective spring of the drill. At step **1540,** and due in part to the compression performed at step **1530,** a drill bit may extend through the protective sleeve and through the implant aperture. Thereafter, at step **1550,** the end user may operably rotate a drive shaft of the drill and begin to drill a passageway into a boney surface, for example. In some embodiments, the bone screw aperture may be angled relative to the implant and/or surfaces of the adjacent boney structure. In those embodiments, due to the conically tapered sleeve portion being seated in an angled bone screw aperture, the passageway may be drilled at an angle that corresponds to the angle of the bone screw aperture. For example, the passageway and bone screw aperture may have the same, similar, or substantially the same angle as measured with respect to the implant and/or boney surface. At step **1560,** the end user may install a bone screw through the bone screw aperture and into the previously drilled passageway at a predefined angle. Furthermore, an end user may, for example, utilize the screwdriver and bone screws disclosed in U.S. Pat. App. No. 17/123,906, titled Screwdriver and Complimentary Screws, filed December 16, 2020, of which this application is a continuation in part of, in conjunction with or as part of a method or system for providing a drilled passage for use with bone screws and a bone screw driver such as that disclosed.

## Claims

1. A drill (100) extending from a distal end (100b) to a proximal end (100a), comprising:
a housing (110) extending in a longitudinal direction;
a rotatable drive shaft (102) including a first drive end (102a) disposed at the proximal end (100a) of the drill (100) and being configured for coupling to a driver, the rotatable drive shaft (102) having a main shaft portion (102b) extending in the longitudinal direction through the housing (110) between the first drive end (102a) and a second drive end (102c);
an angled tip region (106) defining the distal end (100b) of the drill (100), the angled tip region (106) comprising a drill bit coupler (111) configured to receive a drill bit (109) and orient the drill bit (109) in an angled direction with respect to the longitudinal direction thereby defining a drilling axis of the drill bit (109);
a mechanism (103) configured to transfer a rotational force applied to the first drive end (102a) through the second drive end (102c) and drill bit coupler (111); and
a sleeve (108) radially disposed at a distal end of the angled tip region and configured to radially surround at least a first portion of the drill bit (109) when received in the drill bit coupler (111),
**characterized in that** the angled tip region (106) further comprises a compressible spring (107) contacting the sleeve (108) and configured to bias the sleeve (108) in the angled direction,
wherein the compressible spring (107) is configured to surround at least a second portion of the drill bit (109) when received in the drill bit coupler (111).

2. The drill (100) of claim 1, further comprising a positioning handle coupled (104) to and disposed at a medial portion of the housing (110).

3. The drill (100) of claim 2, wherein the positioning handle (140) is angled with respect to the longitudinal direction and extends towards the proximal end (100a) of the drill (100).

4. The drill (100) of claim 1, wherein the sleeve (108) comprises a conically tapered shape.

5. The drill (100) of claim 1, wherein, in a first mode of operation where the spring (107) is in a neutral position, the sleeve (108) and compressible spring (107) are configured to surround lateral sidewalls of the drill bit (109) when received in the drill bit coupler (111).

6. The drill (100) of claim 5, wherein, in a second mode of operation, the compressible spring (107) is configured to compress in a direction parallel to the angled direction towards the mechanism (103).

7. The drill (100) of claim 5, wherein, in a second mode of operation, the compressible spring (107) is configured to compress in a direction parallel to the angled direction towards the mechanism (103) thereby exposing a tip of the drill bit (109) for drilling.

8. The drill (100) of claim 7, wherein, in the first mode of operation where the spring (107) is in a neutral position, the sleeve (108) and compressible spring (107) completely surround the lateral sidewalls of the drill (109).

9. The drill (100) of claim 8, wherein, in the first mode of operation where the spring (107) is in a neutral position, a distal most end of the sleeve (108) extends beyond a distal most end of the drill bit (109).

10. The drill (100) of claim 9, further comprising a flushing hole (112) adjacent the angled tip region (106) and including a flushing path to the mechanism (103).

11. The drill (100) of claim 1, wherein the mechanism (103) is a geared mechanism further comprising:
a first group of teeth (103a) disposed proximate the second drive end (102c); and
a second group of teeth (103b) meshed with the first group of teeth (103a) and extending in a direction parallel with respect to the angled tip portion (106),
wherein the first group of teeth (103a) are meshed with the second group of teeth (103b) to thereby transfer a rotational force applied at the first drive end (102a) to the drill bit coupler (111).

12. The drill (100) of claim 1, wherein said driver comprises a manual hand driver (300) configured to operably couple with the first drive end (102a) of the drive shaft (102).

13. The drill (100) of claim 1, wherein said driver comprises a powered driver (400) configured to operably couple with the first drive end (102a) of the drive shaft (102).

## Patentansprüche

1. Bohrer (100), der sich von einem distalen Ende (100b) zu einem proximalen Ende (100a) erstreckt, umfassend:
ein Gehäuse (110), das sich in einer Längsrichtung erstreckt;
eine drehbare Antriebswelle (102), die ein erstes Antriebsende (102a) einschließt, das an dem proximalen Ende (100a) des Bohrers (100) angeordnet und zum Koppeln mit einem Antrieb konfiguriert ist, wobei die drehbare Antriebswelle (102) einen Hauptwellenabschnitt (102b) aufweist, der sich in der Längsrichtung durch das Gehäuse (110) zwischen dem ersten Antriebsende (102a) und einem zweiten Antriebsende (102c) erstreckt;
einen abgewinkelten Spitzenbereich (106), der das distale Ende (100b) des Bohrers (100) definiert, der abgewinkelte Spitzenbereich (106) umfassend einen Bohreinsatzkoppler (111), der konfiguriert ist, um einen Bohreinsatz (109) aufzunehmen und den Bohreinsatz (109) in einer abgewinkelten Richtung in Bezug auf die Längsrichtung auszurichten, wodurch eine Bohrachse des Bohreinsatzes (109) definiert wird;
einen Mechanismus (103), der konfiguriert ist, um eine Drehkraft, die auf das erste Antriebsende (102a) ausgeübt wird, über das zweite Antriebsende (102c) und den Bohreinsatzkoppler (111) zu übertragen; und
eine Hülse (108), die an einem distalen Ende des abgewinkelten Spitzenbereichs radial angeordnet und konfiguriert ist, um mindestens einen ersten Abschnitt des Bohreinsatzes (109) radial zu umgeben, wenn in dem Bohreinsatzkoppler (111) aufgenommen,
**dadurch gekennzeichnet, dass** der abgewinkelte Spitzenbereich (106) ferner eine komprimierbare Feder (107) umfasst, die die Hülse (108) berührt und konfiguriert ist, um die Hülse (108) in die abgewinkelte Richtung vorzuspannen,
wobei die komprimierbare Feder (107) konfiguriert ist, um mindestens einen zweiten Abschnitt des Bohreinsatzes (109) zu umgeben, wenn in dem Bohreinsatzkoppler (111) aufgenommen.

2. Bohrer (100) nach Anspruch 1, ferner umfassend einen Positioniergriff, der mit einem mittleren Abschnitt des Gehäuses (110) gekoppelt (104) und an diesem angeordnet ist.

3. Bohrer (100) nach Anspruch 2, wobei der Positioniergriff (140) in Bezug auf die Längsrichtung abgewinkelt ist und sich zu dem proximalen Ende (100a) des Bohrers (100) hin erstreckt.

4. Bohrer (100) nach Anspruch 1, wobei die Hülse (108) eine konisch verjüngte Form umfasst.

5. Bohrer (100) nach Anspruch 1, wobei, in einem ersten Betriebsmodus, in dem sich die Feder (107) in einer Nullstellung befindet, die Hülse (108) und die komprimierbare Feder (107) konfiguriert sind, um laterale Seitenwände des Bohreinsatzes (109) zu umgeben, wenn in dem Bohreinsatzkoppler (111) aufgenommen.

6. Bohrer (100) nach Anspruch 5, wobei, in einem zweiten Betriebsmodus, die komprimierbare Feder (107) konfiguriert ist, um in einer Richtung parallel zu der abgewinkelten Richtung zu dem Mechanismus (103) hin zu komprimieren.

7. Bohrer (100) nach Anspruch 5, wobei, in einem zweiten Betriebsmodus, die komprimierbare Feder (107) konfiguriert ist, um sich in einer Richtung parallel zu der abgewinkelten Richtung zu dem Mechanismus (103) hin zu komprimieren, wodurch eine Spitze des Bohreinsatzes (109) zum Bohren freigelegt wird.

8. Bohrer (100) nach Anspruch 7, wobei, in dem ersten Betriebsmodus, in dem sich die Feder (107) in einer Nullstellung befindet, die Hülse (108) und die komprimierbare Feder (107) die lateralen Seitenwände des Bohrers (109) vollständig umgeben.

9. Bohrer (100) nach Anspruch 8, wobei, in dem ersten Betriebsmodus, in dem sich die Feder (107) in einer Nullstellung befindet, ein distalstes Ende der Hülse (108) über ein distalstes Ende des Bohreinsatzes (109) hinaus erstreckt.

10. Bohrer (100) nach Anspruch 9, ferner umfassend ein Spülloch (112) angrenzend an den abgewinkelten Spitzenbereich (106) und einschließlich eines Spülwegs zu dem Mechanismus (103).

11. Bohrer (100) nach Anspruch 1, wobei der Mechanismus (103) ein Getriebemechanismus ist, ferner umfassend:
eine erste Gruppe von Zähnen (103a), die nahe dem zweiten Antriebsende (102c) angeordnet ist; und
eine zweite Gruppe von Zähnen (103b), die mit der ersten Gruppe von Zähnen (103a) in Eingriff steht und sich in einer Richtung parallel in Bezug auf den abgewinkelten Spitzenabschnitt (106) erstreckt,
wobei die erste Gruppe von Zähnen (103a) mit der zweiten Gruppe von Zähnen (103b) in Eingriff steht, um dadurch eine Drehkraft, die auf das erste Antriebsende (102a) ausgeübt wird, auf den Bohreinsatzkoppler (111) zu übertragen.

12. Bohrer (100) nach Anspruch 1, wobei der Antrieb einen manuellen Handantrieb (300) umfasst, der konfiguriert ist, um mit dem ersten Antriebsende (102a) der Antriebswelle (102) wirkgekoppelt zu werden.

13. Bohrer (100) nach Anspruch 1, wobei der Antrieb einen leistungsbetätigten Antrieb (400) umfasst, der konfiguriert ist, um mit dem ersten Antriebsende (102a) der Antriebswelle (102) wirkgekoppelt zu werden.

## Revendications

1. Perceuse (100) s'étendant d'une extrémité distale (100b) à une extrémité proximale (100a), comprenant :
un boîtier (110) s'étendant dans une direction longitudinale ;
un arbre d'entraînement rotatif (102) comprenant une première extrémité d'entraînement (102a) disposée à l'extrémité proximale (100a) de la perceuse (100) et étant conçue pour être couplée à un organe d'entraînement, l'arbre d'entraînement rotatif (102) ayant une partie d'arbre principale (102b) s'étendant dans la direction longitudinale à travers le boîtier (110) entre la première extrémité d'entraînement (102a) et une seconde extrémité d'entraînement (102c) ;
une région de pointe inclinée (106) définissant l'extrémité distale (100b) de la perceuse (100), la région de pointe inclinée (106) comprenant un dispositif d'accouplement de perceuse (111) conçu pour recevoir une perceuse (109) et orienter la perceuse (109) dans une direction inclinée par rapport à la direction longitudinale, définissant ainsi un axe de perçage de la perceuse (109) ;
un mécanisme (103) conçu pour transférer une force de rotation appliquée à la première extrémité d'entraînement (102a) par l'intermédiaire de la seconde extrémité d'entraînement (102c) et du dispositif d'accouplement de perceuse (111) ; et
un manchon (108) disposé radialement à une extrémité distale de la région de pointe coudée et conçu pour entourer radialement au moins une première partie de la perceuse (109) lorsqu'il est reçu dans le dispositif d'accouplement de perceuse (111),
**caractérisée en ce que** la région de pointe inclinée (106) comprend en outre un ressort compressible (107) en contact avec le manchon (108) et conçu pour solliciter le manchon (108) dans la direction inclinée,
dans laquelle le ressort compressible (107) est conçu pour entourer au moins une seconde partie de la perceuse (109) lorsqu'il est reçu dans le dispositif d'accouplement de perceuse (111).

2. Perceuse (100) selon la revendication 1, comprenant en outre une poignée de positionnement couplée (104) à une partie médiane du boîtier (110) et disposée au niveau de celle-ci.

3. Perceuse (100) selon la revendication 2, dans laquelle la poignée de positionnement (140) est inclinée par rapport à la direction longitudinale et s'étend vers l'extrémité proximale (100a) de la perceuse (100).

4. Perceuse (100) selon la revendication 1, dans laquelle le manchon (108) comprend une forme coniquement effilée.

5. Perceuse (100) selon la revendication 1, dans laquelle, dans un premier mode de fonctionnement où le ressort (107) est dans une position neutre, le manchon (108) et le ressort compressible (107) sont conçus pour entourer les parois latérales de la perceuse (109) lorsqu'il est reçu dans le dispositif d'accouplement de perceuse (111).

6. Perceuse (100) selon la revendication 5, dans laquelle, dans un second mode de fonctionnement, le ressort compressible (107) est conçu pour se comprimer dans une direction parallèle à la direction inclinée vers le mécanisme (103).

7. Perceuse (100) selon la revendication 5, dans laquelle, dans un second mode de fonctionnement, le ressort compressible (107) est conçu pour se comprimer dans une direction parallèle à la direction inclinée vers le mécanisme (103), exposant ainsi une pointe de la perceuse (109) pour le perçage.

8. Perceuse (100) selon la revendication 7, dans laquelle, dans le premier mode de fonctionnement où le ressort (107) est dans une position neutre, le manchon (108) et le ressort compressible (107) entourent complètement les parois latérales de la perceuse (109).

9. Perceuse (100) selon la revendication 8, dans laquelle, dans le premier mode de fonctionnement où le ressort (107) est dans une position neutre, une extrémité la plus distale du manchon (108) s'étend au-delà d'une extrémité la plus distale de la perceuse (109).

10. Perceuse (100) selon la revendication 9, comprenant en outre un trou de rinçage (112) adjacent à la région de pointe inclinée (106) et comprenant un passage de rinçage vers le mécanisme (103).

11. Perceuse (100) selon la revendication 1, dans laquelle le mécanisme (103) est un mécanisme à engrenages comprenant en outre :
un premier groupe de dents (103a) disposé à proximité de la seconde extrémité d'entraînement (102c) ; et
un second groupe de dents (103b) engrené avec le premier groupe de dents (103a) et s'étendant dans une direction parallèle par rapport à la partie de pointe inclinée (106),
dans laquelle le premier groupe de dents (103a) est en prise avec le second groupe de dents (103b) pour transférer ainsi une force de rotation appliquée à la première extrémité d'entraînement (102a) au dispositif d'accouplement de perceuse (111).

12. Perceuse (100) selon la revendication 1, dans laquelle ledit dispositif d'entraînement comprend un dispositif d'entraînement manuel (300) conçu pour s'accoupler de manière fonctionnelle avec la première extrémité d'entraînement (102a) de l'arbre d'entraînement (102).

13. Perceuse (100) selon la revendication 1, dans laquelle ledit dispositif d'entraînement comprend un dispositif d'entraînement motorisé (400) conçu pour s'accoupler de manière fonctionnelle avec la première extrémité d'entraînement (102a) de l'arbre d'entraînement (102).
